Europäisches Patentamt

(19) European Patent Office

Office européen des brevets

(11) Publication number: **0 152 694
B1**

(12) **EUROPEAN PATENT SPECIFICATION**

(45) Date of publication of patent specification: **11.04.90**

(21) Application number: **84308706.5**

(22) Date of filing: **13.12.84**

(51) Int. Cl.⁵: **A 61 M 16/04**

(54) **Tracheal tubes.**

(30) Priority: **21.02.84 US 582064**

(43) Date of publication of application:
**28.08.85 Bulletin 85/35**

(45) Publication of the grant of the patent:
**11.04.90 Bulletin 90/15**

(84) Designated Contracting States:
**AT BE CH DE FR GB IT LI LU NL SE**

(56) References cited:
**EP-A-0 072 230
GB-A- 693 510
US-A-3 481 339
US-A-4 417 576**

(73) Proprietor: **BIVONA, INC.
5700 West 23rd Avenue
Gary Indiana, 46406 (US)**

(72) Inventor: **Shapiro, Seymour W.
6400 West 152nd Court
Lowell Indiana 46356 (US)**

(74) Representative: **Newby, Martin John et al
J.Y. & G.W. Johnson Furnival House 14-18 High
Holborn
London WC1V 6DE (GB)**

Courier Press, Leamington Spa, England.

## Description

The present invention relates to tracheal tubes and, more particularly, to tracheal tubes of the type embodying a cuff for effecting a seal between the tube and a trachea.

As is well known in the art, tracheal tubes are commonly inserted into a person's trachea for various purposes; for example, to enable the person to breathe, or to enable intermittent positive pressure ventilation of the respiratory tract to be carried out. The tracheal tube of the present invention is of the cuffed type and is particularly well adapted for the mechanical ventilation of the respiratory tract, especially where high ventilating pressures are utilized.

Tracheal tubes, as that term is used herein, may be of different types, such as, for example, orotracheal tubes, nasotracheal tubes and tracheostomy tubes. Typically, such tubes include a main body portion in the form of an elongated tube made of flexible material such as, rubber polyvinylchloride, or they may be stiff or rigid, made of material such as stainless steel.

There are two types of cuffed tracheal tubes commonly used in the art. One type of tracheal tube is characterized by a cuff including a cover filled with resilient material, with the cover normally being disposed in expanded position and being collapsed by applying a vacuum thereto during insertion or removal of the tube into or from the trachea. A tracheal tube of this general type is described in U.S. Patent No. 3,640,282, issued to Kamen and Wilkinson on August 6, 1970, and forms the subject matter of our co-pending application EP—A—0072230.

A second type of tracheal tube is characterized by a cuff which is not filled with a resilient material. Such cuffs are uninflated or deflated in condition. With such devices, after the intubation device has been inserted into the trachea, the cuff is inflated like a balloon, by feeding air or other working fluid thereinto at a positive pressure to thereby expand the cuff into engagement with the inner wall of the trachea. However, it has been found that such devices have several inherent disadvantgages, the primary disadvantage being that they commonly cause injury to the trachea, causing lesions such as tracheal stenosis, tracheal malacia and localized erosion, particularly if it is necessary for the tube to remain in the trachea for prolonged periods of time.

A known tracheal tube of the inflatable/deflatable type is disclosed in US—A—3481339 and has a cuff provided with an inner chamber and a surrounding outer chamber.

Current techniques of mechanical ventilation of the respiratory tract often employ relatively high positive air pressure. This pressure is highest at the peak of the inspiratory phase and lowest at the termination of the expiratory phase. The human trachea is elastic and stretches increasing in diameter, in part, with increasing pressure in the respiratory tract. The degree of elasticity of the trachea varies and is dependent upon a number of factors, the majority of which cannot be controlled during mechanical ventilation.

Mechanical ventilation techniques and tracheal elasticity create a problem for cuffed endotracheal tubes to overcome. If the cuff is of the air-filled type, the volume of air required to prevent leakage of ventilation gases at peak inspiratory airway pressure exceeds the volume of air needed in the cuff during expiration. The cuff must be overinflated during expiration to prevent leakage during inspiration. The result in many instances is a progressive stretching of the trachea with ultimate tracheal injury.

On the other hand, when the cuff on the tracheal tube is of the expandable-material filled type, such as, for example, the type disclosed in the aforementioned Kamen and Wilkinson U.S. Patent No. 3,640,282, the cuff contents exert progressively less force against the tracheal wall as the elastic trachea's volume is expanded at peak inspiratory pressure. If this expansion of the elastic trachea is of sufficient magnitude, the result is an inadequate seal between the cuff and the trachea.

Heretofore, in an attempt to ensure the proper seal between the trachea and a cuffed tracheal tube during mechanical ventilation and to minimize or prevent tracheal injury, cuff inflator machines have been used. These machines either attempt to vary the cuff volume and pressure synchronously with a companion ventilating machine or to maintain a constant cuff pressure while varying the cuff volume during the changing requirements of a complete inspiration/expiration cycle. These machines have several disadvantages, among which are that they are expensive and are subject to the maintenance and calibration problems inherent with precision machinery.

The present invention seeks to provide a novel tracheal tube which is particularly useful during high positive pressure ventilation of a respiratory tract. The present invention also aims to provide an effective seal between the tracheal tube and the trachea, without the peak pressure in the cuff exceeding the peak pressure of the airway passage. The controlled pressure in the cuff provides a tracheal tube which is more comfortable and minimizes injuries to the trachea.

According to the present invention there is provided a tracheal tube as claimed in the ensuing Claim 1.

Thus, the present invention provides an apparatus which prevents the peak pressure in the cuff from exceeding peak airway pressures and is practical, simple, and efficient in operation and can be readily and economically practiced. Other features and advantages of the present invention will be apparent from the following description and claims.

An embodiment of the invention will now be described, by way of example, with reference to the accompanying drawings, in which,

Figure 1 is a side elevational view of a tracheal tube embodying the principles of the present invention;

Figure 2 is a fragmentary, side elevational view of a portion of the tracheal tube shown in Figure 1, showing parts thereof disposed in a different operative position;

Figure 3 is a cross-sectional view of the tracheal tube shown in Figure 1, looking in the direction of the arrows 3—3 in Figure 1;

Figure 4 is a fragmentary elevational view of the tracheal tube shown in Figure 1, showing the cuff disposed in a trachea in an expanded position, the trachea being shown diagrammatically:

Figure 5 is a view similar to Figure 4 showing the cuff in substantially uninflated or deflated condition; and,

Figure 6 is a fragmentary view of a tracheal tube of the type shown in Figure 1, illustrating a modified form of the present invention.

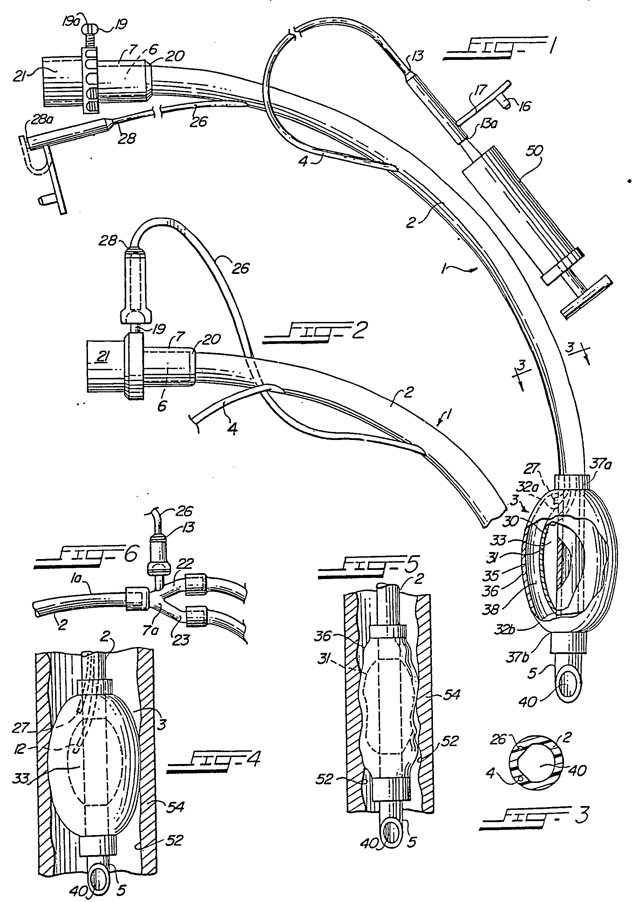

A tracheal tube or intubation device, generally designated by numeral 1, embodying the principles of the present invention, is shown in the drawings to illustrate what is now considered the best mode or preferred embodiment. Referring specifically to Figure 1, the tracheal tube 1 includes the following major elements: an elongated tube 2, and a cuff 3 having an inner inflatable balloon or member 30 and an outer inflatable balloon or member 35.

The elongated tube 2 defines a passage 40 along its length for the purpose of feeding air, or the like, into and out of respiratory tract of a patient into whose trachea the trachea tube 1 has been inserted. When the tracheal tube 1 is to be used as an endotracheal tube, such as either an orotracheal or nasotracheal tube, the elongated tube 2 is preferably expandable and may be made of any suitable material such as rubber, polyvinylchloride, or the like. However, in other instances, such as when the tracheal tube 1 is to be used as a tracheostomy tube, it may be desired to have the elongated tube 2 be rigid in construction and made of a suitable material such as stainless steel.

The elongated tube 2 has a distal end 5 for insertion into the trachea, and a proximal end 6 on which a connector 7 is mounted for connecting the elongated tube 2 to suitable sources of gases for respiration or anesthesia, or to a ventilating machine. It will be understood by those skilled in the art that the elongated tube 2 may be of any suitable length, such tubes commonly being in the nature of 22.5—35.5 cm in length, when used as an endotracheal tube, and commonly being considerably shorter when used as a tracheostomy tube.

Cuff 3 will normally be disposed closer to the distal end 5 of the elongated tube 2 than to the proximal end 6. Normally, cuff 3 is spaced from the distal end portion 5 a distance in the range of approximately 1.25 cm to 1.90 cm (½ inch—¾ inch) on an elongated tube 2 having an overall length of 35.5 cm (14 inches). However, as will be appreciated by those skilled in the art, the cuff 3 may be disposed at any suitable location along the elongated tube 2, the particular location thereof depending upon the intended use of the intubation device 1. For example, the above range would be appropriate if the elongated tube 2 is to be inserted into the windpipe or trachea only. However, if the intubation device 1 is to be inserted further into the trachea, such as for example, into the bronchia, the cuff 3 preferably would be spaced a greater distance from the distal end 5 so that it would remain in the trachea when the distal end 5 is inserted into the bronchia.

Cuff 3, encircling a portion of the outer surface of the elongated tube 2, includes an inner inflatable balloon or member 30 and an outer inflatable balloon or member 35. The inner inflatable balloon or member 30 includes an expandable cylindrical covering 31 disposed on the elongated tube 2 in surrounding relationship. The edge surfaces 32a and 32b of the cylindrical covering 31 are secured to the outer surfaces of the elongated tube 2 in a substantially airtight manner to define an inner chamber 33, as shown in Figure 1.

The outer inflatable balloon or member 35 includes an expandable cylindrical covering 36 disposed on the elongated tube 2 over the inner member 30 in surrounding relationship. The edge surfaces 37a and 37b of the expandable cylindrical covering 36 are secured to the outer surfaces of the elongated tube 2 in a substantially airtight manner to define an outer chamber 38.

The expandable cylindrical coverings 31 and 36 of the inner and outer members 30 and 35, respectively, may be made of any suitable material such as latex rubber or a suitable plastic sheet material, such as polyvinylchloride.

Means for inflating the inner member 30 include an inner inflation tube 4 having a distal end portion 12 and a proximal end portion 13. Inner inflation tube 4 extends into the inner chamber 33 in the space between the cylindrical covering 31 of inner member 30 and the elongated tube 2, at its distal end portion 12, as shown in Figures 1 and 4.

Means for inflating the outer member 35 include an outer inflation tube 26 having a distal end portion 27 and a proximal end portion 28. Outer inflation tube 26 extends into the outer chamber 38 in the space between the cylindrical covering 36 of the outer member 35 and the cylindrical covering 31 of the inner member 30, at its distal end portion 27, as shown in Figures 1 and 4.

Portions of the inner and outer inflation tubes 4 and 26 can be formed integrally with the elongated tube 2 with proximal end portions 13 and 28 projecting outwardly from the proximal end 6 of elongated tube 2, as can best be seen in Figures 1 and 3. However, as will be appreciated by those skilled in the art, either or both of the inner or outer inflation tubes 4 and 26 may be formed separately from the elongated tube 2, inserted and sealably affixed into the respective inner and outer chambers 33 and 38 in a suitable manner without departing from the broader aspects of the present invention.

Inner inflation tube 4 is shown connected at its proximal end 13 to a pump 50 suitable for inflating inner member 30 with air. After the inner member 30 has been inflated, the proximal portion 13 of inner inflation tube 4 is pinched to close the passage within tube 4 and to allow the removal of pump 50 without loss of air. A stopper 16 is affixed to the proximal end portion 13 by means of an expandable strap 17. Stopper 16 can be fitted into the opening 13a of the inner inflation tube 4 to secure the inflation of inner member 30 in a semi-permanent manner. In use, the inner balloon member 30 is inflated by pump 50 to seal against the walls 52 of the trachea 54, when the trachea is at its smallest volume, i.e. the position as shown in Figure 5. This provides a minimum volume seal of the trachea.

The outer inflation tube 26 may be used to inflate outer member 35. Outer inflation tube 26 is also equipped with a stopper 16 affixed to an expandable strap 17 which can be manually bent over to fit the stopper 16 into the opening 28a of outer inflation tube 26 to maintain a positive pressure within the interior of outer balloon member 35.

However, when used with mechanical ventilation of the respiratory tract, it is preferred that the pressure within the outer member 35 fluctuates with the pressure of the respiratory tract of airway pressure within airway passage 40. Thus, an embodiment of the present invention includes the outer chamber 38 in communication with the interior or airway passage 40 of the elongated tube 2 for subjecting the outer chamber 38 of the outer inflatable balloon member 35 to substantially the same pressure as the interior of the elongated tube and, consequently, to the pressure of the airway passage 40 or respiratory tract. Referring now to Figure 2, connector 7 has one end portion 20 connected to the proximal end portion 6 of the elongated tube 2 and another end portion 21 projecting outwardly therefrom for connection to sources of respiration gases, anesthesia and the like or to a mechanical ventilation machine. A coupling 19 is embodied in the connector 7 as an integral part thereof projecting laterally outward from the body of the connector 7 and adapted to receive the proximal end portion 28 of the outer inflation tube 26. Coupling 19 includes a passageway 19a in communication with the interior of the connector 7 and the airway passage 40 of the elongated tube 2.

As will be appreciated by those skilled in the art, the coupling 19 is shown herein as part of the connector 7 merely by way of illustrating one of the preferred forms of the present invention and not by way of limitation, the coupling 19 may be connected to the elongated tube 2 in other ways, such as directly to the elongated tube 2 as an integral part thereof.

Another embodiment is illustrated in Figure 6, wherein identical parts which are the same as are those shown in Figures 1—5 are indicated by the same reference numerals, and parts which are similar to but have been substituted for parts shown in Figures 1—5 are shown by the same reference numerals with the suffix a added. Tracheal tube 1a shown in Figure 6 is of the same construction as the tracheal tube 1 shown in Figures 1—5 except that a modified form of connector 7a has been substituted for the connector 7. Connector 7a has an end portion 20 in communication with the proximal end portion 6 of the elongated tube 2. The end portion 21a of the connector 7a is not of a one peice construction but embodies two branches 22 and 23 by which the tracheal tube 1a may be connected to two separate lines or hoses for connecting the patient to a source of respiratory gases such as oxygen or general anesthetic or the like.

Normally, during mechanical ventilation, gases having a relative humidity of one hundred percent are delivered to the lungs. In most instances, moisture in the cuff 3 is undesirable in that it condenses and forms an uncomfortable weight in the trachea and distorts the shape of the cuff. To protect against this, if desired, a moisture absorbing cartridge, not shown, may be interposed between the coupling 19 and proximal end of the portion 13 of outer inflation tube 38. This cartridge may be of any suitable type readily available on the market, but preferable should be of the type that changes color when it becomes moisture laden so as to alert those in attendance that a replacement cartridge is needed.

In operation, the tracheal tube is inserted into the trachea of a patient with the inner and outer balloon members 30 and 35 deflated and presenting a compact streamlined profile. After the tracheal tube 1 is in place, pump 50 attached to inner inflation tube 4 inflates inner member 30 to seal the trachea while the trachea is in its smallest diameter, generally corresponding to low ventilation pressures, as shown in Figure 5. The proximal end portion 13 of tube 4 is pinched, blocking the passage of gas from the inner member 30 while stopper 16 is pressed firmly within the opening of the proximal end portion 13 of the inner inflation tube 4. Thus, the inner balloon serves to prevent aspiration when the airway pressure is low and trachea 54 is at its smallest volume or diameter.

End portion 21 of connector 7 is coupled in communication to a mechanical ventilation machine (not shown). Air being forced through the elongated tube 2 inflates the respiratory tract and causes the trachea to expand elastically. Outer inflation tube 26 is connected in communication with elongated tube 2 by connecting proximal end portion 28 of the outer inflation tube 26 to coupling 19 of connector 7. Positive pressure in the elongated tube 2 forces the inflation of the outer member 35 at a pressure corresponding to the pressure exerted upon the respiratory tract and the trachea, as shown in Figure 4. Therefore, outer member 35 expands to seal the trachea at a pressure corresponding to the overall pressure in the respiratory tract, and contracts as the pressure falls.

Thus, while the preferred embodiments of the present invention have been illustrated and described, it is to be understood that these are capable of variation and modification, and the present invention should not be limited to the precise details set forth but should include such changes and alterations as fall within the purview of the following claims.

**Claims**

1. A tracheal tube (1) comprising an elongate tubular member (2) a cuff mounted on the tubular member (2) and including an inner inflatable member (30) affixed to, and encircling a portion of, the elongate tubular member (2) to define a first inflation chamber (33) between the tubular member and the inner inflatable member (30), an outer inflatable member (35) surrounding the inner inflatable member (30) to define a second inflation chamber (38) between the inner and outer inflatable members (30, 35), and inflating means (4, 26) including first inflation means (4) for inflating the inner inflatable member and second inflation means in the form of an inflation tube (26) having a distal end which opens into the second inflation chamber (38) for controlling the pressure in the second inflation chamber (38), characterised in that the first inflation means comprises a further inflation tube (4) provided with means for closing off its interior passage thus enabling the inner inflatable member (30) to be inflated to and retained at a desired inflation pressure at which the inflatable cuff seals against the walls of the trachea when the trachea is at its smallest diameter, and in that the first-mentioned inflation tube (26) has a proximal end which is capable of being placed in communication with the interior of the elongate tubular member (2) so that in use the pressure in the second inflation chamber (38) fluctuates in dependence on the pressure in the elongate tubular member (2).

2. A tracheal tube according to Claim 1, characterised by connecting means (7) at a proximal end portion of said tubular member (2) remote from said cuff and having a coupling (19) in communication with the interior of said elongate tubular member (2), the said proximal end of the said first-mentioned inflation tube being connectible to said coupling (19) to place the interior of said tubular member (2) in communication with said second inflation chamber (38).

3. A tracheal tube according to Claim 2, characterised in that the said first-mentioned inflation tube (26) includes means (28) adjacent its proximal end for connecting the first-mentioned inflation tube to the said coupling (19).

4. A tracheal tube according to Claim 1, 2 or 3, characterised in that the said further inflation tube (4) has a proximal end portion (13) and a distal end portion (12), the distal end portion of the said further inflation tube being in communication with said first inflation chamber (33), and the proximal end portion of the said further inflation tube including means (13) for connecting said further inflation tube (4) to a source of pressure.

5. A tracheal tube according to any one of the preceding claims, characterised in that said inner inflatable member (30) includes an expansible tubular covering (31) having edge surfaces (32a, 32b), said expansible tubular covering encircling a portion of said tubular member (2), said edge surfaces of said tubular covering being secured to the outer surface of said tubular member in a substantially air tight manner to define said first inflation chamber (33).

6. A tracheal tube according to any one of the preceding claims, characterised in that said outer inflatable member (35) includes an expansible tubular covering (36) having edge surfaces (37a, 37b) said expansible tubular covering encircling a portion of said tubular member (2) and extending over said inner inflatable member (30) in surrounding relationship, said edge surfaces being secured to the outer surface of said tubular member in a substantially air tight manner to define said second inflation chamber (38).

**Patentansprüche**

1. Trachealtubus (1), bestehend aus einem länglichen rohrförmigen Glied (2), einer auf dem rohrförmigen Glied (2) angebrachten und ein inneres aufblasbares Glied (30), das an dem länglichen rohrförmigen Glied (2) befestigt ist und ein Teil davon umgibt, um eine erste Aufblaskammer (33) zwischen dem rohrförmigen Glied und dem inneren aufblasbaren Glied (30) zu definieren, ein äußeres aufblasbares Glied (35), das das innere aufblasbare Glied (30) umgibt, um eine zweite Aufblaskammer (38) zwischen den inneren und äußeren aufblasbaren Gliedern (30, 35) zu definieren, sowie Aufblasmittel (4, 26) mit ersten Aufblasmitteln (4) zum Aufblasen des inneren aufblasbaren Glieds und zweite Aufblasmittel in Form eines Aufblasrohres (26) mit einem distalen Ende, das zur Kontrolle des Drucks in der zweiten Aufblaskammer (38) in die zweite Aufblaskammer (38) mündet, aufweisende Manschette, dadurch gekennzeichnet, daß das erste Aufblasmittel ein weiteres, mit Mitteln zur Abdichtungs seines inneren Durchgangs ausgestattetes Aufblasrohr (4) umfaßt, wodurch das innere aufblasbare Glied (30) auf einen gewünschten Aufblasdruck, bei dem die aufblasbare Manschette abdichtend gegen die Wände der Luftröhre liegt, wenn die Luftröhre ihren kleinsten Durchmesser aufweist, aufgeblasen und gehalten wird, und daß das erstgenannte Aufblasrohr (26) ein proximales Ende aufweist, das mit dem Inneren des länglichen rohrförmigen Glieds (2) in Verbindung gebracht werden kann, so daß bei Gebrauch der Druck in der zweiten Aufblaskammer (38) abhängig vom Druck im länglichen rohrförmigen Glied (2) schwankt.

2. Trachealtubus nach Anspruch 1, gekennzeichnet durch Verbindungsmittel (7) an einem proximalen Endteil jenes rohrförmigen Glieds (2) entfernt von jener Manschette und mit einer Kupplung (19), die mit dem Inneren jenes länglichen rohrförmigen Gliedes (2) in Verbindung steht, wobei jenes proximale Ende jenes erstgenannten Aufblasrohres mit jener Kupplung (19) verbunden ist, um das Innere jenes rohrförmigen Glieds (2) mit jener zweiten Aufblaskammer (38) in Verbindung zu setzen.

3. Trachealtubus nach Anspruch 2, dadurch gekennzeichnet, daß jenes erstgenannte Aufblasrohr (26) neben seinem proximalen Ende Mittel (28) zur Verbindung des erstgenannten Aufblasrohres mit jener Kupplung (19) einschließt.

4. Trachealtubus nach Anspruch 1, 2 oder 3, dadurch gekennzeichnet, daß jenes weitere Aufblasrohr (4) einen proximalen Endteil (13) und einen distalen Endteil (12) aufweist, wobei der distale Endteil jenes weiteren Aufblasrohres mit jener ersten Aufblaskammer (33) in Verbindung steht, und der proximale Endteil jenes weiteren Aufblasrohres Mittel (13) zur Verbindung jenes weiteren Aufblasrohres (4) mit einer Druckquelle einschließt.

5. Trachealtubus nach einem der vorhergehenden Ansprüche, dadurch gekennzeichnet, daß jenes innere aufblasbare Glied (30) eine ausdehnbare rohrförmige Abdeckung (31) mit Randflächen (32a, 32b) einschließt, wobei jene ausdehnbare rohrförmige Abdeckung einen Teil jenes rohrförmigen Glieds (2) umgibt, und wobei jene Randflächen jener rohrförmigen Abdeckung an der Außenfläche jenes rohrförmigen Glieds in einer im wesentlichen luftdichten Weise befestigt sind, um jene erste Aufblaskammer (3) zu definieren.

6. Trachealtubus nach einem der vorhergehenden Ansprüche, dadurch gekennzeichnet, daß jenes äußere aufblasbare Glied (35) eine ausdehnbare rohrförmige Abdeckung (36) mit Randflächen (37a, 37b) einschließt, wobei jene ausdehnbare rohrförmige Abdeckung einen Teil jenes rohrförmigen Glieds (2) umgibt, und sich über jenes innere aufblasbare Glied (30) in umrundenden Verhältnis erstreckt, wobei jene Randflächen an der Außenfläche jenes rohrförmigen Glieds in einer im wesentlichen luftdichten Weise befestigt sind, um jene zweite Aufblaskammer (38) zu definieren.

**Revendications**

1. Tube trachéal (1) comprenant un long organe tubulaire (2), une manchette montée sur l'organe tubulaire (2) et comportant un organe gonflable intérieur (30) qui est fixé au long organe tubulaire (2) et qui encercle une partie de ce dernier de manière à définir une première chambre de gonflage (33) entre l'organe tubulaire et l'organe gonflable intérieur (30), un organe gonflable extérieur (35) entourant l'organe gonflable intérieur (30) afin de définir une seconde chambre de gonflage (38) entre les organes gonflables intérieur et extérieur (30, 35), et des moyens de gonflage (4, 26) comprenant un premier moyen de gonflage (4) pour gonfler l'organe gonflable intérieur, et un second organe de gonflage ayant la forme d'un tube de gonflage (26) comportant une extrémité distale qui s'ouvre dans la seconde chambre de gonflage (38) pour régler la pression dans cette seconde chambre de gonflage (38), caractérisé en ce que le premier moyen de gonflage comprend un autre tube de gonflage (4) pourvu d'un moyen destiné à obturer son passage intérieur, ce qui permet de gonfler l'organe gonflable intérieur (30) et de le maintenir à une pression de gonflage souhaitée à laquelle la manchette gonflable est appliquée de manière étanche contre les parois de la trachée lorsque celle-ci est à son diamètre minimum, et en ce que le premier tube de gonflage (26) comporte une extrémité proximale qui peut être placée en communication avec l'intérieur du long organe tubulaire (2), de telle sorte qu'en service, la pression dans la seconde chambre de gonflage (38) fluctue en fonction de la pression dans le long organe tubulaire (2).

2. Tube trachéal suivant la revendication 1, caractérisé par un moyen de raccordement (7) à une partie d'extrémité proximale de l'organe tubulaire (2) éloignée de la manchette et comportant un raccord (19) en communication avec l'intérieur du long organe tubulaire (2), l'extrémité proximale du tube de gonflage mentionné en premier lieu pouvant être raccordée au raccord (19) pour mettre l'intérieur de l'organe tubulaire (2) en communication avec la seconde chambre de gonflage (38).

3. Tube trachéal suivant la revendication 2, caractérisé en ce que le tube de gonflage (26) mentionné en premier lieu comprend un moyen (28) près de son extrémité proximale pour le raccorder au raccord (19).

4. Tube trachéal suivant la revendication 1, 2 ou 3, caractérisé en ce que l'autre tube de gonflage (4) comporte une partie d'extrémité proximale (13) et une partie d'extrémité distale (12), la partie d'extrémité distale et cet autre tube de gonflage étant en communication avec la première chambre de gonflage (33) et la partie d'extrémité proximale de cet autre tube de gonflage comprenant un moyen (13) pour raccorder cet autre tube de gonflage (4) à une source de pression.

5. Tube trachéal suivant l'une quelconque des revendications précédentes, caractérisé en ce que l'organe gonflable intérieur (30) comprend un recouvrement tubulaire extensible (31) comportant des surfaces marginales (32a, 32b), le recouvrement tubulaire extensible encerclant une partie de l'organe tubulaire (2), les surfaces marginales du recouvrement tubulaire étant fixées à la surface extérieure de l'organe tubulaire d'une manière en substance étanche à l'air afin de définir la première chambre de gonflage (33).

6. Tube trachéal suivant l'une quelconque des revendications précédentes, caractérisé en ce que l'organe gonflable extérieur (35) comprend un recouvrement tubulaire extensible (36) comportant des surfaces marginales (37a, 37b), le recouvrement tubulaire extensible encerclant une partie de l'organe tubulaire (2) et s'étendant pardessus l'organe gonflable intérieur (30) tout autour de celui-ci, les surfaces marginales étant fixées à la surface extérieure de l'organe tubulaire d'une manière en substance étanche à l'air afin de définir la seconde chambre de gonflage (38).

EP 0 152 694 B1

FIG-1

FIG-2

FIG-6

FIG-5

FIG-4

FIG-3

1